# FASCICULE DE BREVET EUROPEEN

(11) **EP 1 228 198 B1**
(45) Date de publication et mention de la délivrance du brevet: **05.12.2007**
(21) Numéro de dépôt: 00974608.2
(22) Date de dépôt: 30.10.2000
(51) Int. Cl.: C12N 9/78, C12N 15/52

(54) **PROCEDE DE MODULATION DE LA SELECTIVITE DES NITRILASES, NITRILASES OBTENUES PAR CE PROCEDE ET LEUR UTILISATION**
VERFAHREN ZUR SPEZIFIZITÄTSMODULIERUNG VON NITRILASEN, NITRILASEN AUS DIESEM VERFAHREN GEWONNEN UND DEREN VERWENDUNG
METHOD FOR MODULATING NITRILASE SELECTIVITY, NITRILASES OBTAINED BY SAID METHOD AND USE THEREOF

(30) Priorité: 08.11.1999 FR 9914249
(43) Date de publication de la demande: 07.08.2002
(73) Titulaire: Adisseo France S.A.S., 92160 Antony (FR)
(72) Inventeur: PIERRARD, Jérôme, 69370, ST Didier au Mont d'Or (FR); FAVRE-BULLE, Olivier, F-30900 Nimes (FR); JOURDAT, Catherine, F-69110 Sainte-Foy-lès-Lyon (FR)
(74) Mandataire: Guerre, Dominique
(86) Numéro de dépôt international: PCT/FR2000/003028
(87) Numéro de publication internationale: WO 2001/034786

(56) Documents cités:
- KOBAYASHI M ET AL: "NITRILASE IN BIOSYNTHESIS OF THE PLANT HORMONE INDOLE-3-ACETIC ACID FROM INDOLE-3-ACETONITRILE: CLONING OF THE ALCALIGENES GENE AND SITE-DIRECTED MUTAGENESIS OF CYSTEINE RESIDUES" PROCEEDINGS OF THE NATIONAL ACADEMY OF SCIENCES OF USA,US,NATIONAL ACADEMY OF SCIENCE. WASHINGTON, vol. 90, 1 janvier 1993 (1993-01-01), pages 247-251, XP002036846 ISSN: 0027-8424
- KOMEDA H ET AL: "TRANSCRIPTIONAL REGULATION OF THE RHODOCOCCUS RHODOCHROUS J1 NITA GENE ENCODING A NITRILASE" PROCEEDINGS OF THE NATIONAL ACADEMY OF SCIENCES OF USA,US,NATIONAL ACADEMY OF SCIENCE. WASHINGTON, vol. 93, no. 20, 1 octobre 1996 (1996-10-01), pages 10572-10577, XP002045089 ISSN: 0027-8424
- KOBAYASHI M ET AL: "NITRILASE FROM RHDODOCOCCUS RHODOCHROUS J1. SEQUENCING AND OVEREXPRESSION OF THE GENE AND IDENTIFICATION OF AN ESSENTIAL CYSTEINE RESIDUE" JOURNAL OF BIOLOGICAL CHEMISTRY,US,AMERICAN SOCIETY OF BIOLOGICAL CHEMISTS, BALTIMORE, MD, vol. 267, no. 29, 15 octobre 1992 (1992-10-15), pages 20746-20751, XP002031260 ISSN: 0021-9258
- WATANABE A ET AL: "Cloning and expression of a gene encoding cyanidase from Pseudomonas stutzeri AK61." APPLIED MICROBIOLOGY AND BIOTECHNOLOGY, vol. 50, no. 1, juillet 1998 (1998-07), pages 93-97, XP000924971 ISSN: 0175-7598
- WATANABE ATSUSHI ET AL: "Investigation of the potential active site of a cyanide dihydratase using site-directed mutagenesis." BIOCHIMICA ET BIOPHYSICA ACTA, vol. 1382, no. 1, 15 janvier 1998 (1998-01-15), pages 1-4, XP000924835 ISSN: 0006-3002
- LEVY-SCHIL SOPHIE ET AL: "Aliphatic nitrilase from a soil-isolated Comamonas testosteroni sp.: Gene cloning and overexpression, purification and primary structure." GENE (AMSTERDAM), vol. 161, no. 1, 1995, pages 15-20, XP004042111 ISSN: 0378-1119
- NOVO CARLOS ET AL: "Pseudomonas aeruginosa aliphatic amidase is related to the nitrilase/cyanide hydratase enzyme family and Cys-166 is predicted to be the active site nucleophile of the catalytic mechanism." FEBS LETTERS, vol. 367, no. 3, 1995, pages 275-279, XP000915286 ISSN: 0014-5793
- BORK PEER ET AL: "A new family carbon-nitrogen hydrolases." PROTEIN SCIENCE, vol. 3, no. 8, 1994, pages 1344-1346, XP000924973 ISSN: 0961-8368

### LISTE DE SEQUENCES

<110> Aventis Animal Nutrition
<120> Procédé de modulation de la sélectivité des nitrilases, nouvelles nitrilases obtenues par ce procédé et leur utilisation
<130> PH 99069
<140>
   <141>
<150> FR 9914249
   <151> 1999-11-08
<160> 5
<170> PatentIn Ver. 2.1
<210> 1
   <211> 28
   <212> ADN
   <213> Séquence artificielle
<220>
   <223> Description de la séquence artificielle: Oligonucléotide NitB162
<400> 1
   cgcgtcggtg ccctgmastg ctgggagc 28
<210> 2
   <211> 22
   <212> ADN
   <213> Séquence artificielle
<220>
   <223> Description de la séquence artificielle: Oligonucléotide SR
<400> 2
   cggcaatgat caggccttcg gc 22
<210> 3
   <211> 20
   <212> ADN
   <213> Séquence artificielle
<220>
   <223> Description de la séquence artificielle: Oligonucléotide NitB1
<400> 3
   gcagcacagg gcaccgacgc 20
<210> 4
   <211> 34
   <212> ADN
   <213> Séquence artificielle
<220>
   <223> Description de la séquence artificielle: Oligonucléotide NitA163
<400> 4
   gcatgttccc agcagcagag tcccccaaga ttcc 34
<210> 5
   <211> 34
   <212> ADN
   <213> Séquence artificielle
<220>
   <223> Description de la séquence artificielle: Oligonucléotide NitA162
<400> 5
   ggaatcttgg gggactctgc tgctgggaac atgc 34

## Description

La présente invention concerne de nouvelles nitrilases à sélectivité améliorée, le procédé pour les obtenir et l'utilisation desdites nitrilases.

### Les nitrilases

Les enzymes catalysant l'hydrolyse de groupements nitriles en acides carboxyliques correspondants et ions ammoniums sont les nitrilases (Faber, Biotransformations in Organic Chemistry, Springer Verlag, Berlin Heidelberg , 1992, ISBN3-540-55762-8). Toutefois, cette bioconversion des groupements nitriles en acides carboxyliques correspondants, dont le bilan final consiste en une hydrolyse des groupements nitriles peut aussi être réalisée en deux étapes, la première étape consistant en la conversion des nitriles en amides correspondants par une nitrile hydratase, la deuxième étape consistant à hydrolyser les amides obtenus en acides carboxyliques correspondants par des amidases.

Les nitrilases furent découvertes d'abord chez les plantes (Thimann and Mahadevan, 1964, Arch. Biochem. Biophys. 105: 133-141) puis isolées chez de nombreux représentants de la microflore du sol (Kobayashi et Shimizu, 1994, FEMS Microbiology Letters 120: 217-224) : *Pseudomonas, Nocardia, Arthrobacter, Fusarium, Rhodoccacus, Klebsielle, Alcaligenes*. Plus récemment, des nitrilases ont été caractérisées chez des bactéries thermophiles (Cramp et al., 1997, Microbiology, 143 : 2313-2320). Les nitrilases ont des spécificités de substrat variées mais peuvent être regroupees en trois groupes en fonction de leur spécificité : les nitrilases spécifiques des nitriles aliphatiques, celles spécifiques de nitriles aromatiques ou celles spécifiques d'arylacétonitriles (Kobayashi et al., 1993, Proc. Natl. Acad. Sci. USA 90: 247-251 ; Kobayashi et Shimizu, 1994, précité : Lévy-Schil et al., 1995, Gene 161 : 15-20 ; Layh et al., 1998, J. Mol. Catal B : Enzymatic 5 : 467-474).

Les nitrilases ont un intérêt en biocatalyse car de nombreux procédés de synthèse mettent en jeu l'hydrolyse de groupements nitriles (Yamamoto et al., 1991, Appl. Environ. Microb. 57: 3028-3032; Faber, Biotransformations in Organic Chemistry, 2nd edn, Springer-Vertag, Berlin, 1995 ; Lévy-Schil et al., 1995, Gene 161 : 15-20 ; Cowan et al., 1998, Extremophiles 2 : 207-216): conversion de l'adiponitrile en cyanovalérate ou adipate, synthèse de l'acide nicotinique, de l'acide p-aminobenzoïpue, de l'acide tranexamique, hydrolyse énantiosélective de mandelonitrile. En particulier, la nitrilase *d'Alcaligenes faecalis* ATCC8750 (appelée dans cette demande NitB) et celle de *Comamonas testosteroni* (appelée dans cette demande NitA) peuvent être utilisées pour accéder à l'nydroxy-analogue de la méthionine (FR9411301, WO9609403, FR9613077).

Les nitrilases ont des structures primaires qui s'alignent avec des degrès d'identité variables à partir d'environ 30%. Un alignement des séquences de plusiseurs nitrilases fait apparaître la conservation de plusieurs résidus dont un résidu cystéine en position 163 sur la séquence de la nitrilase de NitB. Ce résidu est impliqué dans le mécanisme réactionnel des nitrilases (Kobayashi et al., 1993, Proc. Natl. Acad. Sci. USA 90 : 247-251).

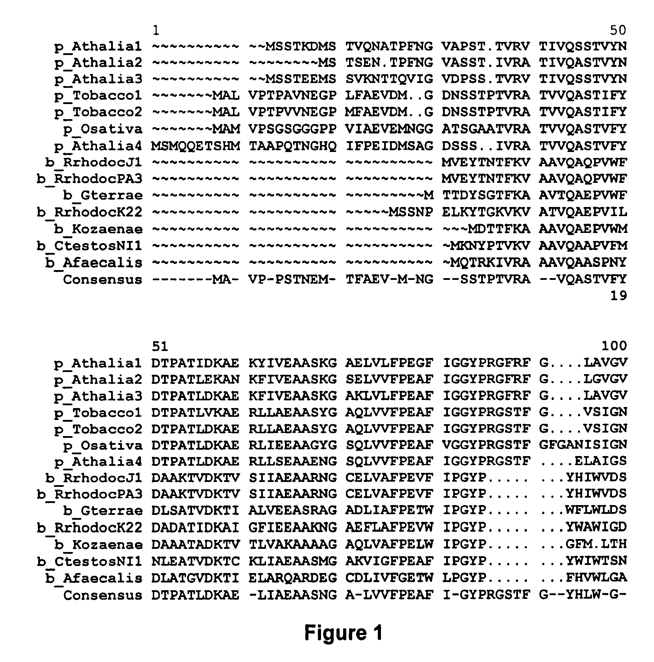

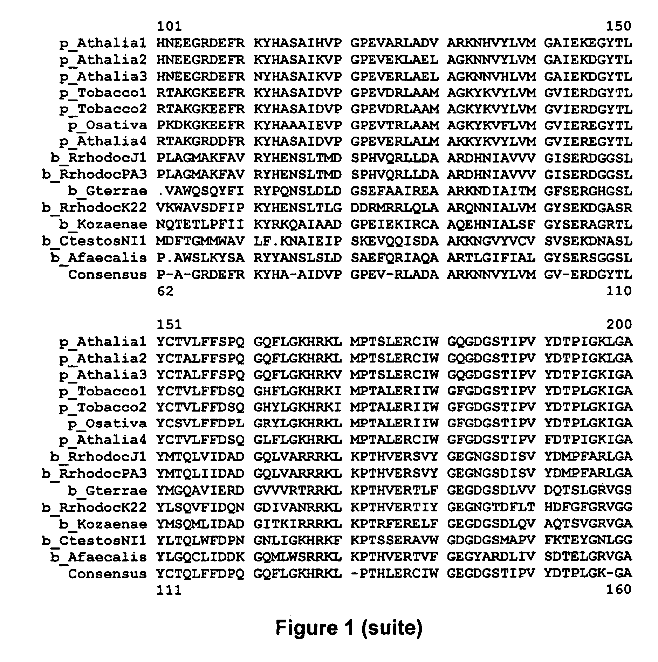

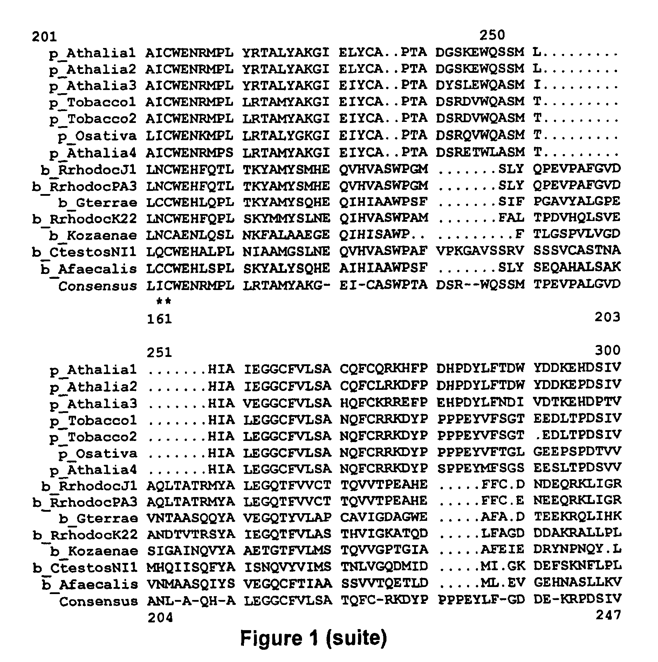

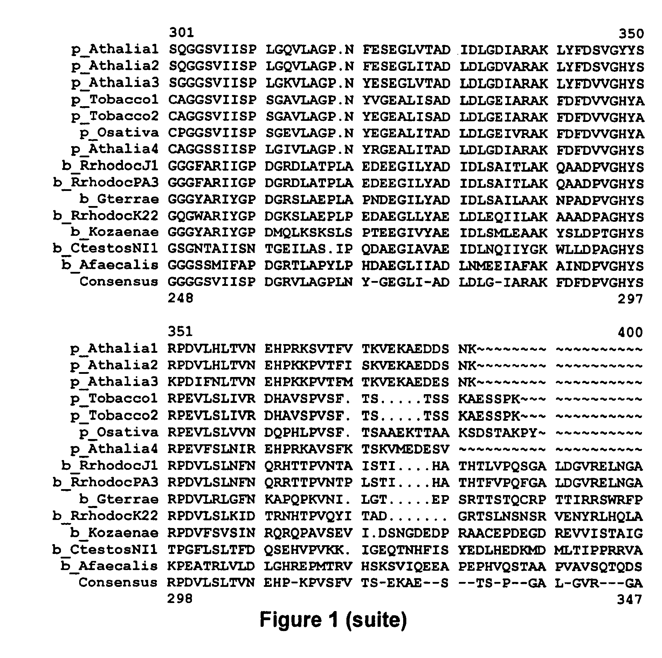

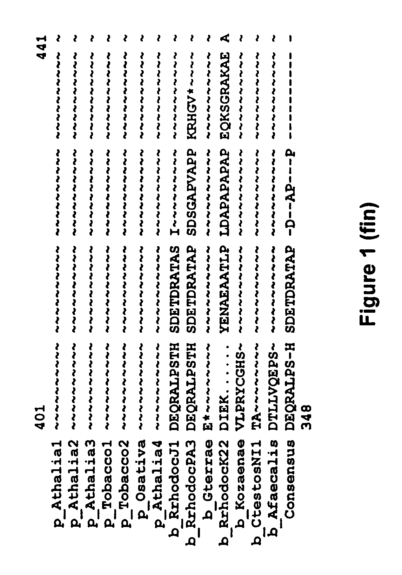

Pour la présente invention, la séquence de référence est la séquence NitB, toutes les définitions et indications de positions d'acides aminés particuliers étant faites par rapport à la séquence primaire NitB. La figure 1 en annexe représente un alignement de 14 séquences de nitrilases décrites dans l'état de la technique, alignées par rapport à la séquence NitB comme référence, comprenant les séquences p_Athalia1 à 4 *d'Arabidopsis thaliana* (N° accession SwissProt: P32961, P32962, P46010, P46011), p_Tobacco1 et 2 de *Nicotiana tabacum* (N° accession GeneBank: D63331, D83078), b_RhodocJ1 de *Rhodococcus rhodocrous* J1 (N° accession GeneBank: D11425), b_RrhococPA3 de *Rhodococcus rhodocrous* PA34 (N° accession GeneBank: E09026), b_Gterrae de *Gordona terrae* (N° accession GeneBank: E12616), b_RrhodocK22 de *Rhodococcus rhodocrous* K22 (N° accession GeneBank: D12583), b_Kozaenae de *Klebsielia ozaenae* (N° accession SwissProt: P100450), b_CtestosNI1 de *Comamonas testosteroni NI1* ou NitA (N° accession GeneBank: L32589). et b_Afaecalis d'*Alcaligenes faecalis* JM3 ou NitB (N° accession SwissProt: P20960). La numérotation des acides aminés de la séquence de NitB est donnée sur cette figure (numérotation du bas), de même que la séquence consensus avec sa numérotation (numérotation du haut). Par convent on dans cette demande, la numérotation des résidus des autres nitrilases se fait par rapport à ce résidu cystéine et la séquence de la nitrilase NitB comme séquence de référence. Sur la base d'un tel alignement, ou de tout alignement de séquences de nitrilases, il est aisé pour l'homme du métier d'identifier à partir de la définition de l'acide aminé ce NitB par sa position et sa nature, la position de l'acide aminé correspondant dans une autre séquence de nitrilase.

### Le problème de la sélectivité

La sélectivité des nitrilases se définit comme le pourcentage de composés ne possédant pas de fonction carboxylique libérés par l'hydrolyse d'un nitrile catalysée par une nitrilase. Elle a été relativement peu décrite mais s'observe pour un certain nombre de nitrilases et de substrats. Ainsi, l'hydrolyse du 2-(methoxy)-mandelonitrile en acide 2-(methoxy)-mandelique catalysée par la nitrilase de *Peudomonas fluorescens* DSM 7155 conduit à la coproduction de 2-(methoxy)-mandelamide (Layh *et al*., 1998, précité). De même, l'hydrolyse de 2-hydroxy 4-(méthyl thio) butironitrile (HMTBN) catalysé par la nitrilase NitA de *Comamonas testosteroni* NI1 (Lévy-Schil *et al.,* 1995, précité) décrite dans les exemples de cette demande est accompagnée de la co-production de 2-hydroxy 4-méthyl thio butiramide (HMTBM). Dans le cas d'un procédé biocatalytique mettant en jeu un tel couple enzyme/substrat, le manque de sélectivité de la nitrilase pour son substrat conduit à une co-production d'amide qui représente une perte de rendement du procédé. Cette perte de rendement peut avoir un impact économique considérable. De plus, ce manque de sélectivité conduit à la présence d'amide contaminant le produit de la réaction. La purification de l'acide carboxylique est alors nécessaire et, là encore, a un impact économique sur le procédé. Le manque de sélectivité d'une nitrilase pour un substrat donné est par conséquent un obstacle au développement d'un procédé biocatalytique mettant en jeu cette nitrilase et ce substrat.

La diminution de la sélectivité des enzymes peut également être recherchée si celle-ci s'accompagne d'une augmentation de l'activité catalytique de l'enzyme sur son substrat. C'est notamment le cas dans des procédés de décontamination où l'on recherche à dégrader rapidement une molécule toxique avec une enzyme à activité spécifique maximum. Dans ce cas, la nature des produits issus de la réaction catalysée par l'enzyme a peu d'importance relativement à la vitesse de dégradation du substrat.

Il est donc particulièrement important de pouvoir modifier la sélectivité des nitrilases, tant dans le sens d'une amélioration de cette sélectivité que dans le sens d'une diminution.

### L'amélioration des enzymes

L'évolution dirigée d'une enzyme consiste à adapter une enzyme à une fonction particulière en sélectionnant de manière itérative des variants qui présentent des propriétés améliorées (Arnold et Volkov, 1999, Current Opinion in Chemical Biology 3 : 54-59 ; Kuchner et Arnold, 1997, Tibtech 15 : 523-530). Ces variants peuvent être créés par plusieurs techniques de mutagenèse sur le gène codant pour l'enzyme étudiée (Skandalis et al., 1997, Chemistry & Biology 4 : 8889-898 ; Crameri et al., 1998, Nature 391 : 288-291); mutagenèse chimique (Singer et Fraenkel-Conrat, 1969, Prog. Nucl. Acid Res. Mol. Biol. 9 : 1-29), mutagenèse par PCR en conditions d'erreurs (Leung et al., 1989, Technique 1 :11-15), par PCR combinatoire (Crameri *et al*., 1998, précité ; Shao et al., 1698, Nucleic Acids Res. 26: 681-683), par mutagenèse dirigée (Directed Mutagenesis : A Practical Approach, 1991. Edited by M.J. McPHERSON, IRL PRESS), etc.

Dans le cadre d'ur processus d'amélioration de l'activité de la nitrilase NitB sur HMTBN (2-amino-4-(métnylthio)butyronitrile), les inventeurs ont constaté qu'une substitution ponctuelle sur NitB dans la région du site actif en position 162, en remplaçant le résidu cystéine par un résidu asparagine, entraînait une altération de la sélectivité de la nitrilase étudiée. A contrario, Us ont constaté qu'en introduisant un résidu cystéine en position 162 sur la séquence de la nitrilase NitA entraînait une amélioration de sa sélectivité sur un autre de ses substrats, l'AMTBN, démontrant ainsi que la position 162 dans la région du site actif des nitrilases est une position clé impliquée la sélectivité desdites nitrilases et que la modification du résidu acide aminé à cette position consistant à remplacer l'acide aminé d'origine par un autre acide aminé entraîne une modulation de la sélectivité des nitrilases.

La présente invention concerne donc une nitrilase modifiée à sélectivité modulée, caractérisée en ce qu'elle comprend en position 162 un résidu acide aminé différent du résidu acide aminé d'origine.

Plus précisément, la présente invention concerne donc une nitrilase modifiée caractérisée en ce que:
a) l'acide aminé en position 162 de la nitrilase est remplacé par une cystéine, ladite cystéine étant différente de l'acide aminé d'origine de la nitrilase, la position des acides amines étant définie par rapport à la séquence primaire de la nitrilase NitB d'*Alcaligenes faecalis* représentée à la figure 1, et
b) la sélectivité de la nitrilase est améliorée, la sélectivité de la nitrilase étant définie comme le pourcentage de composés, ne possédant pas de fonction carboxylique, libérés par l'hydrolyse d'un nitrile catalysée par la nitrilase,
à l'exception de la nitrilase b_Gterrae de *Gordona terrae* (N° accession GeneBank : E12616) et de la nitrilase b_Afaecalis d'*Alcaligenes faecalis* (N° accession SwissProt : P20960).

Par nitrilase modifiée, on entend selon l'invention une nitrilase modifiée par rapport à une nitrilase d'origine, la modification consistant à remplacer le résidu acide aminé d'origine en position 162 par un autre acide aminé.

Par modulation de la sélectivité, on entend selon l'invention une sélectivité de la nitrilase modifiée différente de la sélectivité de la nitrilase d'origine, en particulier d'au moins 0.5 % par rapport à la nitrilase d'origine, avantageusement d'au moins 1%.

De manière avantageuse, le résidu 162 est remplacé par un acide aminé choisi parmi la cystéine, l'alanine, la valine, l'asparagine, la glutamine, l'isoleucine ou la sérine, étant entendu que le résidu 162 de la nitrilase d'origine est différent d'une cystéine, alanine, valine, asparagine, glutamine, isoleucine ou sérine, respectivement.

De préférence, le résidu 162 est remplacé par un résidu cystéine.

La nitrilase d'origine non modifiée est choisie parmi les nitrilase d'origine bactérienne, levure, fongique, végétale ou animale.

Parmi les ntitrilases d'origine bactérienne, on peut citer notamment les nitrilases suivantes : b_RhodocJ1 de *Rhodococous rhodocrous* J1 (N° accession GeneBank: D11425), b_RrhodocPA3 de *Rhodococous rhodocrous* PA34 (N° accession GeneBank: E09026), b_Gterrae de *Gordona terrae* (N° accession GeneBank: E12616), b_RrhodocK22 de *Rhodococcus rhodocrous* K22 (N° accession GeneBank: D12583), b_Kozaenae de *Klebsiella ozaenae* (N° accession SwissProt: P100450), b_CtestosNI1 de *Comamonas testosteroni* NI1 ou NitA (N° accession GeneBank: L32589), et b_Afaecalis d'*Alcaligenes faecalis* JM3 ou NitB (N° accession SwissProt: P20960). Parmi les nitrilase d'origine végétale on peut citer notamment : p_Athalia1 à 4 d'*Arabidopsis thaliana* (N° accession SwissProt: P32961, P32962, P46010, P46011), p_Tobacco1 et 2 de *Nicotiana tabacum* (N° accession GeneBank: D63331, D83078).

Parmi les nitrilases d'autres origines on peut citer notamment: celles *Saccharomyces cerevisiae* (N° d'accession Swiss-Prot P40447 et P4044), de *Caenorhabditis elegans* (N° d'accession GeneBank AF069986), de *Drosophila melanogaster* (GeneBank AF069989), d'*Homo sapiens* (N° d'accession GeneBank AF069987), de *Mus musculus* (N° d'accession GeneBank AF069988).

Selon un autre mode de réalisation de l'invention la nitrilase d'origine est une nitrilase obtenue par criblage de banques d'ADN, notamment ADNc ou ADN génomique de différentes sources, notamment de banques d'ADN obtenues par recombinaisons et mutations aléatoires de nitrilases, par évolution moléculaire dirigée ou par criblage d'une banque d'ADN du sol ou d'autres biotopes.

La présente invention concerne également une séquence d'acide nucléique, en particulier une séquence d'ADN codant pour une nitrilase modifiée ci-dessus.

Selon un premier mode de réalisation de l'invention, la séquence d'acide nucléique selon l'invention est constituée par la séquence d'acide nucléique de la nitrilase d'origine, pour laquelle le codon du résidu d'origine en position 162 a été remplacé par un codon codant pour un résidu différent du résidu d'origine, en particulier les codons codant pour les résidus alanine, valine, asparagine, glutamine, isoleucine ou sérine.

La modification des codons de la séquence d'origine peut être effectuée par tout moyen connu de l'homme du métier d'amélioration des enzymes définis auparavant, en particulier par mutagenèse dirigée.

La présente invention concerne également un gène chimére ou cassette d'expression, comprenant dans le sens de la transcription une séquence de régulation promotrice fonctionnelle dans un organisme hôte, la séquence d'acide nucléique codant pour une nitrilase modifiée selon l'invention et une séquence de régulation terminatrice fonctionelle dans le même organisme hôte.

L'organisme hôte comprend tout organisme eucaryote ou procaryote, différencié ou indifférencié, en particulier les bactéries, les levures, les champignons, les cellules végétales et les plantes.

Il s'agit en particulier de bactéries, par exemple *E. coli*, de levures, en particulier des genres *Saccharomyces, Kluyveromyces* ou *Pichia*, de champignons, en particulier des genres *Aspergillus* ou *Penicilium,* d'un baculovirus, ou encore des cellules végétales et des plantes.

Par "cellule végétale", on entend selon l'invention toute cellule issue d'une plante et pouvant constituer des tissus indifférenciés tels que des cals, des tissus différenciés tels que des embryons, des parties de plantes, des plantes ou des semences.

On entend par "plante" selon l'invention, tout organisme multicellulaire différencié capable de photosynthèse, en particulier monocotylédones ou dicotylédones, plus particulierement des plantes de culture destinées ou non à l'alimentation animale ou humaine, comme le maïs, le blé, le colza, le soja, le riz, la canne à sucre, la betterave, le tabac, le coton, etc.

Les éléments de régulation promotrice et terminatrice sont bien connus de l'homme du métier en fonction des organismes hôtes.

Comme séquence de régulation promotrice dans les bactéries, on peut employer toute séquence de régulation promotrice d'un gène s'exprimant naturellement dans les bactéries, par exemple le promoteur de l'opéron tryptophane d'*E. coli* (Denèfle et al., 1987, Gene 56: 61-70).

Comme séquence de régulation promotrice dans les levures, on peut employer toute séquence de régulation promotrice d'un gène s'exprimant naturellement dans les levures, par exemple le promoteur du gène Mfα1 de *S. cerevisiae*, ou du gène de lactase de *Kluyveromyces lactis* (van den Berg et al., 1990. Bio/Technology 8: 135-139).

Comme séquence de régulation promotrice dans les champignons, on peut employer toute séquence de régulation promotrice d'un gène s'exprimant naturellement dans les champignons, par exemple la séquence promotrice du gène de la phosphatase acide de *Penicillium chrysogenum* (Graessle et al., 1997, Appl. Environ. Mocrobiol. 63:753-756) ou la séquence promotrice du gène de l'alcool deshydrogénase *d'Aspergillus nidulans* (Gwyne et al., 1989, Biochem. Soc. Trans. 17: 338-340).

Comme séquence de régulation promotrice dans les cellules végétales et les plantes, on peut utiliser toute séquence promotrice d'un gène s'exprimant naturellement dans les plantes en particulier un promoteur d'origine bactérienne, virale ou végétale tel que, par exemple, celui d'un gène de la petite sous-unité de ribulose-biscarboxylase/oxygénase (RuBisCO), un promoteur d'histone (EP 0 507 698) ou un promoteur d'actine de riz, ou d'un gène de virus de plante tel que, par exemple, celui de la mosaïque du choux-fleurs (CAMV 19S ou 35S), ou un promoteur inductible par les pathogènes comme le PR-la du tabac, tout promoteur convenable connu pouvant être utilisé.

Comme séquence de régulation terminatrice dans les bactéries, on peut employer toute séquence de régulation terminatrice d'un gène s'exprimant naturellement dans les bactéries, par exemple la séquence de régulation terminatrice de l'opéron des ARN ribosomaux d'*E. coli* (Denèfle et al., 1987, Gene 56: 61-70).

Comme séquence de régulation terminatrice dans les levures, on peut employer toute séquence de régulation terminatrice d'un gène s'exprimant naturellement dans les levures, par exemple le terminateur de la phosphoglycèrate kinase (PGK) de S. *cerevisiae*, ou le terminateur de la lactase *Kluyveromyces lactis* (van den Berg et al., 1990, Bio/Technology 8: 135-139).

Comme séquence de régulation terminatrice dans les champignons, on peut employer toute séquence de régulation terminatrice d'un gène s'exprimant naturellement dans les champignons, par exemple la séquence de régulation terminatrice du gène de la pyruvate kinase de *Trichoderma reesei* (Schindler et al., 1993, Gene 130: 271-275).

Comme séquence de régulation terminatrice dans les cellules végétales ou les plantes, on peut utiliser toute séquence de régulation terminatrice d'un gène s'exprimant naturellement dans les plantes, par exemple le terminateur d'un gène d'origine bactérienne, comme par exemple le terminateur nos d'*Agrobacterium tumefaciens*, d'origine virale, comme par exemple le terminateur du CaMV 35S, ou encore d'origine végétale, comme par exemple un terminateur d'histone (EP 0 633 317).

La présente invention concerne également un organisme hôte transformé comprenant un gène chimère tel que défini ci-dessus, notamment un organisme hôte défini ci-dessus dans le genome duquel le gène chimère selon l'invention a été intégré de manière stable.

La présente invention concerne également un procédé de production de nitrilases modifiées à sélectivité améliorée définies ci-dessus, ledit procédé consistant à cultiver l'organisme hôte transformé selon l'invention, et le cas échéant à isoler la nitrilase modifiée, en mélange ou sous une forme purifiée.

La présente invention concerne également l'utilisation d'une nitrilase modifiée selon l'invention dans une réaction de biocatalyse dans un procédé de synthèse ou de dégradation de composés chimiques.

La presente invention concerne enfin un procédé pour améliorer la sélectivité d'une nitrilase, la sélectivité de la nitrilase étant définie comme le pourcentage de composés, ne possédant pas de fonction carboxylique, libérés par l'hydrolyse d'un nitrile catalysée par la nitrilase, caractérisé en ce qu'il consiste à remplacer l'acide aminé en position 162 de la nitrilase par une cystéine, ladite cystéine étant différente de l'acide aminé d'origine de la nitrilase, la position des acides aminés étant définie par rapport à la séquence primaire de la nitrilase NitB d'*Alcaligenes faecalis* représentée à la figure 1.

De manière avantageuse, le remplacement du résidu 162 est obtenu en introduisant dans la séquence d'acide nucléique codant pour la nitrilase d'origine non modifiée un codon codant pour un résidu à la position 162 différent du codon codant pour le résidu de la nitrilase d'origine.

De préférence, la nitrilase obtenue par ledit procédé de modulation est une nitrilase telle que définie précédemment.

Les exemples ci-après permettent d'illustrer la présente invention, sans chercher à en limiter la portée.

### Matériel et méthodes

La mesure de l'activité nitrilase sur le 2-hydroxy 4-(méthyl thio) butinonitrile est réalisée comme suit :
Un échantillon de culture de densité optique à 660 nm (DO₆₆₀) connue est prélevé et lavé dans du tampon phosphate 100 mM pH 7,0. En estimant le poids sec de l'échantillon d'après la DO₆₆₀ (une unité DO₆₆₀ correspond à un poids sec de 0,35 mg de Cellules Sèches (ml) environ 1 mg de CS est repris dans 1 ml de tampon phosphate 100 mM pH 7,0 et incubé dans un tube fermé de 2 ml à 35°C pendant 10 minutes. La cinétique est démarrée en ajoutant 17 µl de la solution d'HMTBN à 78% pour atteindre la concentration de 100 mM dans le mélange réactionnel au début de l'essai. La réaction est incubée à 35°C sous agitation. Toutes les 15 minutes, un échantillon de 100 µl de la suspension est retiré et mélangé à 900 µl de H₃PO₄ 100 mM pH2,5 pour arrêter la réaction. Après centrifugation, le surnageant est analysé par HPLC comme décrit ci-dessous

L'éluant est composé d'acétonitrile de qualité HPLC dilué dans de l' H₃PO₄ 50 mM (0,9 litre d'H₃PO₄ 50 mM mélangés à 0,1 litre d'acetonitrile). Cet éluant, filtré et dégazé, percole la colonne avec un débit de 1 ml/min et une pression de 140 bar. La colonne utilisée est une colonne ce Nucleosil 5µm C18 de 250 mm de longueur et 4,6 mm de diamètre (INTERCHIM. Ref. N5CC18-25F). Le volume injecté est de 5 µl et la détection est réalisée par lecture de l'absorbance à une longueur d'onde de 215 nm. Dans ces conditions, les pics d'HMTBM, d'HMTBS (acide 2-hydroxy 4-(methyl thio) butanoïque) et d'HMTBN (2-amino-4(méthylthio)butanamide) ont des temps de rétention respectifs de 9 min, 11 min et 15,8 min. Les quantités d'HMTBN, l'HMTBM et l'HMTBS sont déduites de la mesure la surface des pics et de la comparaison à la surface des pics d'un mélange d'étalonnage de composition connue.

La mesure de l'activité nitrilase sur le 2-amino 4-(méthyl thio) butinonitrile est réalisée comme suit :

Un culot cellulaire (entre 0,4 et 20 mg de CS) est resuspendu dans du tampon borate 200 mM pH 9,2 et incubé dans un tube fermé de 2 ml à 30°C pendant 10 minutes. La cinétique est démarrée en ajoutant une solution d'AMTBN afin d'obtenir une concentration final de 50 mM dans le mélange réactionnel au début de l'essai. La réaction est incubée à 30°C sous agitation. Toutes les 15 minutes, un échantillon de 50 µl de la suspension est retiré et mélangé à 950 µl de l'éluant d'HPLC (voir composition ci-dessous) pour arrêter la réaction. Après centrifugation, le surnageant est analysé par HPLC comme décrit ci-dessous.

L'éluant est composé de 1% d'acétonitrile de qualité HPLC dilué dans une solution d' H₃PO₄ à 0,5% dans l'eau. Cet éluant, filtré et dégazé, percole la colonne avec un débit de 1 ml/min La colonne utilisée est une colonne de Nucleosil 5µm C18 de 250 mm de longueur et 4,6 mm de diamètre (INTERCHIM. Ref. N5CC18-25F) maintenue à une température de 40°C. Le volume injecté est de 20 µl et la détection est réalisée par lecture de l'absorbance à une longueur d'onde de 210 nm. Dans ces conditions, les pics d'AMTBM, d'AMTBS (acide 2-amino 4-(methyl thio) butanoïque) et d'AMTBN ont des temps de rétention respectifs de 4,0 min, 4,5 min et 5,0 min. Les quantités d'AMTBN, l'AMTBM et l'AMTBS sont déduites de la mesure de la surface des pics et de la comparaison à la surface des pics d'un mélange d'étalonnage de composition connue.

Les autres techniques mises en oeuvre sont des techniques classiques de biologie moléculaire et de microbiologie, connues de l'homme de l'art et décrites par exemple par Ausubel et ai., 1987 (Current Protocols in Molecular Biology, John Willey and Sons, New York). Maniatis et al., 1982, (Molecular Cloning : a laboratory manual. Cold Spring Harbor Laboratory, Cold Sring Harbor, New York), Coligan et al., 1997 (Current Protocols in Protein Science, John Wiley & Sons, Inc).

### Exemples

### Exemple 1: Construction des plasmides d'expression pBCAT29 et pBCAT41.

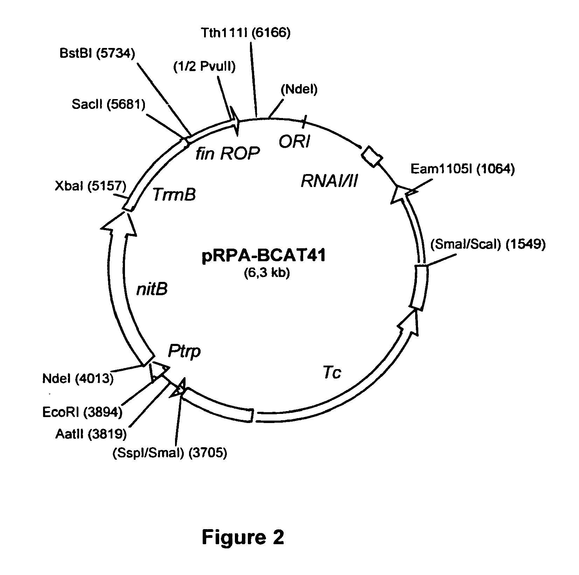

La figure 2 représente la carte du plasmide pRPA-BCAT41. Les sites entre parenthèse sont des sites qui ont été éliminés lors des clonages. Ptrp : promoteur tryptophane nitB : gène de la nitrilase ; TrmB : terminateurs de transcription ; fin ROP : fin du gène codant pour la protéine ROP (Chambers et al., 1988, Gene 68 : 139-149) ; ORI : origine de réplication ; RNAI/II : ARN impliqués dans la réplication ( Chambers *et al*., précité); Tc : gène de résistance à la tétracycline.

Le fragment de 1.27 kb contant le promoteur *Pₜᵣₚ*, le site de fixation du ribosome du gène cII du phage λ (RBScll) et le gène de la nitrilase d'*Alcaligenes faecalis* ATCC8750 (*nitB*) a été extrait du plasmide pRPA68CAT6 (Demande FR 96/13077) à l'aide des enzymes de restriction EcoRI et Xbal pour être cloné dans le vecteur pXL642 (décrit dans la demande CIP N°08/194,588) ouvert par les mêmes enzymes de restriction. Le plasmide résultant, pRPA-BCAT15 a été ouvert par les enzymes StuI et Bsml et le fragment de 4,3 kb a été ligaturé avec le fragment Stul-Bsml de 136 bp purifié de pRPA-BCAT4 (Demande FR 96/13077) pour conduire au plasmide pRPA-BCAT19. Le séquençage partiel de pRPA-BCAT19 a confirmé le remplacement du codon du résidu Asp279 de la nitrilase par le codon d'un résidu Asn279. Le fragment de 1,2 kb EcoRI-XbaI de pRPA-BCAT19 contenant la fusion *Pₜᵣₚ* : :RBScII : :*nitB* a été ensuite cloné dans le vecteur pRPA-BCAT28 ouvert par les mêmes enzymes pour conduire au plasmide pRPA-BCAT29 de 6,2 kb. Le vecteur pRPA-BCAT28 a été obtenu en ligaturant le fragment de 3,9 kb Sspl-Scal de pXL642 (demande CIP N°08/194,588) avec le fragment de 2,1 kb SmaI de pHP45ΩTc (Fellay et al., 1987, Gene 52 : 147-154) afin de remplacer le marqueur de résistance à l'ampicilline par le marqueur de résistance à la tétracycline. En détruisant le site Ndel proche de l'origine de réplication du plasmide pRPA-BCAT29 par digestion partielle NdeI et action de la Polymérase I d'*E. coli* (Fragment de Klenow), le plasmide pRPA-BCAT41 a été obtenu dont une carte est représentée sur la figure 1. En éliminant du plasmide pRPA-BCAT41 le fragment Xcm1 de 0,55 bp par digestion Xcm1 et ligation, le plasmide pRPA-BCAT72 a été obtenu.

### Exemple 2 : Replacement de la cystéine162 de NitB par une asparagine

Le codon de la cystéine 162 du gène *nitB* a été modifié par mutagenèse dirigée en utilisant les amorces NitB162 (CGCGTCGGTGCCCTGMASTGCTGGGAGC où M = A/C S = G/C) et SR (CGGCAATGATCAGGCCTTCGGC).

Un fragment interne de 361 bp a été amplifié par réaction de PCR en utilisant les amorces NitB162 et SR, la matrice pRPA-BCAT41, la polymérase Pwo (Boehringer) et le programmé d'incubation suivant : 5 min à 95°C, cinq cycles (une minute à 95°C, 1 min à 58°C, 1 à 72°C), 35 cycles (45 secondes à 95°C, 30 secondes à 58°C, 30 secondes à 72°C). 5 min à 72°C.

Après purification du fragment amplifié par migration sur gel d'agarose, extraction du fragment à l'aide du kit « QiaEx» (Quiagen), l'ADN a été incubé en présence des enzymes restriction StuI et BanI (New England Biolabs) à 37°C pendant 16h dans un tampon recommandé par le fournisseur.
Un autre fragment interne de 498 bp a été amplifié de façon analogue à l'aide des amorces PCRAF1 (décrite dans la demande FR96/13072) et NitB1 (GCAGCACAGGGCACCGACGC).

Après purification du fragment amplifié par migration sur gel d'agarose, extraction du fragment à l'aide du kit « QiaEx» (Quiagen), l'ADN a été incubé en présence des enzymes de restriction NdeI et BanI (New England Biolabs) à 37°C pendant 16h dans un tampon recommandé par le fournisseur.

Le vecteur pRPA-BCAT72 a été ouvert par les enzymes Ndel et StuI et le fragment de 5,43 kp a été purifié sur gel d'agarose et extrait à l'aide du kit QiaEx. Les trois fragmentes décrits ci-dessus ont alors été ligaturés et le mélange de ligation a été introduit par éléctroporation dans la souche d'E. coli DH5alpha. Les clones obtenus ont été analysés par restriction avec les enzymes EcoRI et Xbal afin de sélectionner des plasmides présentant un insert de 1.26 kb. Après séquençage de la région englobant le codon 162 du gène de la nitrilase, 2 plasmides porteurs du codon recherché (AAC au lieu de TGC) ont été retenus et appelés pRPA-BCAT75.

Ces plasmides ont été introduits dans la souche d'E. coli RPA-BIOCAT496 pour donner les souches RPA-BIOCAT526 et 527. La souche RPA-BIOCAT496 correspond à la souche W (ATCC9637) dans laquelle le plasmide pRPA-BCAT34 a été préalablement introduit Le plasmide pRPA-BCAT34 correspond au plasmide pXL2035 (Lévy-Schil et al., 1995. Gene 161 : 15-20) dans lequel a été cloné entre les sites EcoRI et NotI un fragment de 475 bp porteur du gène *trpR* codant pour le régulateur du promoteur Ptrp. Ce fragment a été extrait du plasmide pRPA-BCAT30, construit par clonage dans le vecteur pSL301 (Brosius, 1989, DNA 8 : 759-777) d'un fragment Aatll-Stul de 434 bp porteur du gène *trpR* et de son promoteur extrait du plasmide pRPG9 (Gunsalus et Yanofsky. 1980, Proc. NatI. Acad. Sci. USA 77 : 7117-7121).

### Exemple 3 : Influence de la cystéine 162 de NitB sur la sé/ectivité de la nitrilase lors de l'hydrolyse de l'HMTBN

Les souches RPA-BIOCAT526, RPA-BIOCAT527 et RPA-BIOCAT 497 (correspondant à la souche RPA-BIOCAT496 dans laquelle le plasmide pRPA-BCAT41 a préalablement été introduit) ont été cultivées dans les conditions décrites dans l'exemple 5 de la demande FR96/13072 avec les modifications suivantes : préculture de 8h. ensemencement au 1:50 dans du milieu M9 glucose contenant 0,4% de casaminoacides, 12 µg/ml de tetracycline et 50 µg/ml de kanamycine.

L'activité nitrilasique sur HMTBN a été mesurée sur un culot cellulaire lavé dans du tampon phosphate de potassium 100 mM pH 7 comme décrit ci-dessus, en engageant 1 mg de CS dans un volume réactionnel de 1 ml. La sélectivité des souches a été mesurée après 2 heures d'hydrolyse en rapportant la surface du pic d'amide formé à la somme des surfaces des pics d'amide et d'acide formés. Elle est exprimée en pourcentage. Les résultats sont présentés dans le tableau 1 :

**Tableau 1 : Sélectivité des souches 526, 527 et 497**

| RPA-BIOCAT | Activité | Sélectivité |
|---|---|---|
| | (µmole/h.mgCS¹) | |
| 497 | 65 | 0,04 % |
| 526 | 76 | 0,2 % |
| 527 | 65 | 0,2% |

| | | |
|---|---|---|
| ¹ mg CS : poids en cellules sèches estimé d'après la densité optique de l'échantillon à 660 nm OD₆₆₀ (mg CS = OD₆₆₀ x 0.35) | | |

Ces résultats montrent que la substitution de la cystéine 162 par une asparagine altère la sélectivité de NitB pour l'hydrolyse de l'HMTBN.

### Exemple 4 : Remplacement de la glutamine 162 de NitA par une cystéine

Le codon de la glutamine 162 de la nitrilase NitA a été modifié en un codon cystéine par mutagenèse dirigée à l'aide du kit QuickChange™ site-directed mutagenesis (Stratagene).

Les amorces NitA163 (GCATGTTCCCAGCAGCAGAGTCCCCCAAGATTCC) et NitA162 (GGAATCTTGGGGGACTCTGCTGCTGGGAACATGC) ont été utilisées dans les conditions préconisées par le fournisseur sur de l'ADN du plasmide pXL2158 (Lévy-Schil et al., 1995 : Gene 161 : 15-20).

Le programme d'incubation de la réaction de PCR comprenait 30 secondes à 95°C et 16 cycles de 30 secondes à 95°C - 1 min à 55°C - 12 minutes à 68°C. Après digestion de l'ADN par DpnI et transformation d'E. coli XL1-Blue par 1 µl du mélange réactionnel, les clones obtenus ont été analysés par profil de restriction plasmidique à l'aide de l'enzyme BpmI. La mutation introduite altérant un site de restriction Bpm1, cinq clones ayant perdu un des 3 sites BpmI ont été sélectionnés. Les plasmides qu'ils contenaient ont été introduits séparément dans la souche RPA-BIOCAT496 pour donner les souches RPA-BIOCAT570 à RPA-BIOCAT574.

### Exemple 5 : Amélioration de la sélectivité de NitA C162 pour l'hydrolyse de l' HMTBN après substitution par une cystéine en position 162

Le plasmide pXL2158 a été introduit dans la souche RPA-BIOCAT496 pour donner la souche RPA-BIOCAT575. Les souches RPA-BIOCAT570 à RPA-BIOCAT575 ont été cultivées dans les conditions d'expression décrites ci-dessus en remplaçant la tétracycline par de l'ampicilline à 100 µg/ml. L'activité nitrilasique de ces souches a été dosée comme décrit ci-dessus, en engageant 5 mg de cellules (en poids sec estimé d'après la OD₆₆₀ ) dans un volume réactionnel de 1 ml et pendant 1 heure. La sélectivité est mesurée en calculant le rapport de la surface du pic correspondant à l'amide à la surface du pic correspondant à l'acide. Elle est exprimée en pourcentage. Le tableau 2 regroupe les résultats.

**Tableau 2 : Sélectivité des souches RPA-BIOCAT570 à 575 sur HMTBN**

| RPA-BIOCAT | Activité | Sélectivité |
|---|---|---|
| | (mmole/h.g CS) | |
| 575 | 135 | 17% |
| 570 | 8 | 4,3 |
| 571 | 8 | 4,3 |
| 572 | 4 | 4,5 |
| 573 | 7 | 4.4 |
| 574 | 4 | 4,5 |

Ces résultats montrent que la substitution de la glutamine 162 de NitA par une cystéine permet un gain de sélectivité d'un facteur 4 pour l'hydrolyse de l'HMTBN.

### Exemple 6 : Amélioration de la sélectivité de NitA pour l'hydrolyse de l'AMTBN après substitution par une cystéine en position 162

Les souches RPA-BIOCAT570 et RPA-BIOCAT575 ont été cultivées dans les conditions d'expression décrites ci-dessus. L'activité nitrilasique de ces souches a été dosée en utilisant comme substrat le 2-amino 4 (méthyl thio) butanenitrile ou AMTBN (C5H10N2S) à raison de 50 mM dans du tampon borate pH 9,2. Dans un volume réactionnel de 1 ml, l'équivalent de 10 mg de cellules (exprimés en poids sec) de RPA-BIOCAT570 et de 0,4 mg mg de cellules (exprimés en poids sec) de RPA-BIOCAT575 ont été engagés à 30°C pendant 24 h. La production d'AMTBS et d'AMTBA ont été mesurées par HPLC comme décrit ci-dessus et la sélectivité calculée de la façon analogue. Les résultats sont présentés dans le tableau 3.

**Tableau 3 : Sélectivité des souches RPA-BIOCAT570 et 575 sur AMTBN**

| RPA-BIOCAT | Numéro Essai | Activité | TT² à 24 h | Sélectivité |
|---|---|---|---|---|
| | | (mmol/h.g CS)¹ | | |
| 575 | 1 | 43 | 98 | 6.3 |
| 575 | 2 | 50 | 100 | 5,6 |
| 570 | 3 | 1,5 | 100 | 1,0 |
| 570 | 2 | 2,7 | 97 | 0,6 |

| | | | | |
|---|---|---|---|---|
| ¹ Activite mesurée après 30 minutes d'hydrolyse ² TT : (quantité d'AMTBN initiale - quantité d'AMTBN à 24h) / quantité d'AMTBN initiale | | | | |

Ces résultats montrent la substitution de la glutamine 162 de NitA par une cystéine permet un gain de sélectivité d'un facteur 4 sur un autre substrat que l'HMTBN, en l'occurrence l'AMTBN.

### Exemple 8: Autre substitution en position 162 sur NitB et perte de sélectivité sur HMTBN

Par séquençage de la région englobant le codon 162 du gène *nitB* hébergé par les clones issus de la mutagenèse décrite dans l'exemple 2, il a été possible d'identifier des clones porteurs du codon CAG au lieu de TGC en position 162. Les plasmides correspondants ont été appelés pRPA-BCAT78 et portent un insert *nitB* codant pour une nitrilase NitB Q162 dont la cystéine 162 est substituée par une glutamine. Ces plasmides ont été introduits dans la souche d'E. coli RPA-BIOCAT496 pour donner les souches RPA-BIOCAT530 et 531. Les souches RPA-BIOCAT530, RPA-BIOCAT531 et RPA-BIOCAT 497 ont été cultivées dans les conditions décrites dans l'exemple 3. L'activité nitrilasique sur HMTBN a été mesurée sur un culot cellulaire lavé dans du tampon phosphate de potassium 100 mM pH 7 comme décrit dans l'exemple 4 de la demande FR96/13072. en engageant 1 mg de CS dans un volume réactionnel de 1 ml. La sélectivité des souches a été mesurée après 2 heures d'hydrolyse en rapportant la quantité molaire d'amide formé à la somme des quantités molaires d'amide et d'acide formés. Elle est exprimée en pourcentage. Les résultats sont présentés dans le tableau 5.

**Tableau 5 : Sélectivité des souches 530, 531 et 497**

| RPA-BIOCAT | Activité | Sélectivité |
|---|---|---|
| | (µmole/h.mgCS¹) | |
| 497 | 65 | 0.04% |
| 530 | 18 | 0,2% |
| 531 | 22 | 0.2% |

| | | |
|---|---|---|
| ¹ mg CS poids en cellules sèches estimé d'après la densité optique de l'échantillon à 660 nm CD₆₆₀ (mg CS = OD₆₆₀ x 0.35) | | |

Ces résultats montrent que la substitution de la cystéine 162 par une glutamine altère la sélectivité de NitB pour l'hydrolyse de l'HMTBN.

## Revendications

1. Nitrilase modifiée **caractérisée en ce que**:
a) l'acide aminé en position 162 de la nitrilase est remplacé par une cystéine, ladite cystéine étant différente de l'acide aminé d'origine de la nitrilase, la position des acides amines étant définie par rapport à la séquence primaire de la nitrilase NitB d'*Alcaligenes faecalis* représentée à la figure 1, et
b) la sélectivité de la nitrilase est améliorée, la sélectivité de la nitrilase étant définie comme le pourcentage de composés, ne possédant pas de fonction carboxylique, libérés par l'hydrolyse d'un nitrile catalysée par la nitrilase,
à l'exception de la nitrilase b_Gterrae de *Gordona terrae* (N° accession GeneBank : E12616) et de la nitrilase b_Afaecalis d'*Alcaligenes faecalis* (N° accession SwissProt : P20960).

2. Nitrilase modifiée selon la revendication 1 **caractérisée en ce que** la nitrilase dans laquelle l'acide amine en position 162 est remplacé par une cystéine, est choisie parmi les nitrilases p_Athalia 1 à 4 d'*Arabidopsis thaliana*, les nitrilases p_Tobacco 1 et 2 de *Nicotiana tabacum,* la nitrilase b_RhodocJ1 de *Rhodococcus rhodocrous* J1, la nitrilase b_RrhodocPA3 de *Rhodococcus rhodocrous* PA34, la nitrilase b_RrhodocK22 de *Rhodococcus rhodocrous* K22, la nitrilase b_Kozaenae de *Klebsiella ozaenae* et la nitrilase Nit A de *Comamonas testosteroni* décrites à la figure 1.

3. Nitrilase modifiée selon l'une des revendications précédentes **caractérisée en ce qu'**elle consiste en la nitrilase NitA de *Comamonas testosteroni* décrite à la figure 1 dans laquelle l'acide aminé en position 162 est remplacé par une cystéine.

4. Séquence d'acide nucléique, en particulier une séquence d'ADN codant pour une nitrilase modifiée selon l'une des revendications 1-3.

5. Gène chimère ou cassette d'expression, **caractérisé en ce qu'**il comprend dans le sens de la transcription une séquence de régulation promotrice fonctionnelle dans un organisme hôte, la séquence d'acide nucléique selon la revendication 4 codant pour une nitrilase modifiée et une séquence de régulation terminatrice fonctionnelle dans le même organisme hôte.

6. Organisme hôte transformé non humain **caractérisé en ce qu'**il comprend un gène chimère selon la revendication 5 intégré de manière stable dans son génome.

7. Organisme hôte selon la revendication 6 **caractérisé en ce qu'**il est choisi parmi les bactéries, les levures, les champignons, les cellules végétales et les plantes.

8. Procédé de production de nitrilases modifiées selon l'une des revendications 1-3 **caractérisé en ce qu'**il consiste à cultiver un organisme hôte transformé selon l'une des revendications 6-7 et le cas échéant à isoler la nitrilase modifiée, en mélange ou sous forme purifiés.

9. Utilisation d'une nitrilase modifiée selon l'une des revendications 1-3 dans une réaction de biocatalyse dans un procédé de synthèse ou de dégradation de composés chimiques.

10. Utilisation de la nitrilase modifiée selon la revendication 3 pour l'hydrolyse de l'HMTBN ou de l'AMTBN.

11. Procédé pour améliorer la sélectivité d'une nitrilase, la sélectivité de la nitrilase étant définie comme le pourcentage de composés, ne possédant pas de fonction carboxylique, libérés par l'hydrolyse d'un nitrile catalysée par la nitrilase, **caractérisé en ce qu'**il consiste à remplacer l'acide aminé en position 162 de la nitrilase par une cystéine, ladite cystéine étant différente de l'acide aminé d'origine de la nitrilase, la position des acides aminés étant définie par rapport à la séquence primaire de la nitrilase NitB d'*Alcaligenes faecalis* représentée à la figure 1.

12. Procédé selon la revendication 11 **caractérisé que** la nitrilase, dans laquelle l'acide aminé en position 162 est remplacé par une cystéine, est choisie parmi les nitrilases p_Athalia 1à 4 d'*Arabidopsis thaliana,* les nitrilases p_Tobacco 1 et 2 de *Nicotiana tabacum,* la nitrilase b_RhodocJ1 de *Rhodococcus rhodocrous* J1, la nitrilase b_RrhodocPA3 de *Rhodococcus rhodocrous* PA34, la nitrilase b_RrhodocK22 de *Rhodococcus rhodocrous* K22, la nitrilase b_Kozaenac de *Klebsiella ozaenae* et la nitrilase Nit A de *Comamonas testosteroni* décrites à la figure 1.

13. Procédé selon la revendication 12 **caractérisé en ce qu'**il consiste à remplacer l'acide aminé en position 162 par une cystéine dans la nitrilase NitA de *Comamonas testosteroni* décrite à la figure 1.

## Claims

1. A modified nitrilase **characterized in that**:
a) the amino acid in the position 162 of the nitrilase is replaced with cysteine, said cysteine being different from the original amino acid of the nitrilase, the position of the amino acids being defined relatively to the primary sequence of the NitB nitrilase of *Alcaligenes faecalis* illustrated in Fig. 1, and
b) the selectivity of the nitrilase is enhanced, the selectivity of the nitrilase being defined as the percentage of compounds, not having any carboxylic function, released by hydrolysis of a nitrile catalyzed by the nitrilase,
with the exception of the nitrilase b_Gterrae from *Gordona terrae* (GeneBank access No.: E12616) and of the nitrilase b_Afaecalis from *Alcaligenes faecalis* (SwissProt access No.: P20960).

2. The modified nitrilase according to claim 1, **characterized in that** the nitrilase wherein the amino acid in position 162 is replaced with cysteine, is selected from the nitrilases p_Athalia 1 to 4 from *Arabidopsis thaliana,* the nitrilases p_Tobacco 1 and 2 from *Nicotiana tabacum,* the nitrilase b_RhodocJ1 from *Rhodococcus rhodocrous* J1, the nitrilase b_RrhodocPA3 from *Rhodococcus rhodocrous* PA34, the nitrilase b_RrhodocK22 from *Rhodococcus rhodocrous* K22, the nitrilase b_Kozaenae form *Klebsiella ozaenae* and the nitrilase NitA from *Comamonas testosteroni* described in Fig. 1.

3. The modified nitrilase according to any of the preceding claims, **characterized in that** it consists in the nitrilase NitA from *Comamonas testosteroni* described in Fig. 1, wherein the amino acid in position 162 is replaced with cysteine.

4. A nucleic acid sequence, in particular a DNA sequence, coding for a modified nitrilase according to any of claims 1 to 3.

5. A chimeric gene or an expression cassette, **characterized in that** it comprises in the direction of the transcription, a functional promoter regulatory sequence in a host organism, the nucleic acid sequence according to claim 4 coding for a modified nitrilase and a functional terminator regulatory sequence in the same host organism.

6. A non-human transformed host organism **characterized in that** it comprises a chimeric gene according to claim 5 integrated in a stable way into its genome.

7. The host organism according to claim 6, **characterized in that** it is selected from bacteria, yeasts, fungi, vegetable cells and plants.

8. A method for producing modified nitrilases according to any of claims 1 to 3, **characterized in that** it consists of cultivating a transformed host organism according to any of claims 6 to 7 and if necessary, isolating the modified nitrilase, either as a mixture or in purified form.

9. The use of a modified nitrilase according to any of claims 1 to 3 in a biocatalysis reaction in a method for synthesis or degradation of chemical compounds.

10. The use of the modified nitrilase according to claim 3 for hydrolysis of HMTBN or AMTBN.

11. A method for improving the selectivity of a nitrilase, the selectivity of the nitrilase being defined as the percentage of compounds, not having any carboxylic function, released by hydrolysis of a nitrile, catalyzed by the nitrilase, **characterized in that** it consists of replacing the amino acid in position 162 of the nitrilase with cysteine, said cysteine being different from the original amino acid of the nitrilase, the position of the amino acids being defined relatively to the primary sequence of the nitrilase NitB from *Alcaligenes faecalis* illustrated in Fig. 1.

12. The method according to claim 11, **characterized in that** the nitrilase, wherein the amino acid in position 162 is replaced with cysteine, is selected from the nitrilases p_Athalia 1 to 4 from *Arabidopsis thaliana,* the nitrilases p_Tobacco 1 and 2 from *Nicotiana tabacum,* the nitrilase b_RhodocJ1 from *Rhodococcus rhodocrous* J1, the nitrilase b_RrhodocPA3 from *Rhodococcus rhodocrous* PA34, the nitrilase b_RrhodocK22 from *Rhodococcus rhodocrous* K22, the nitrilase b_Kozaenae form *Klebsiella ozaenae* and the nitrilase NitA from *Comamonas testosteroni* described in Fig. 1.

13. The method according to claim 12, **characterized in that** it consists of replacing the amino acid in position 162 with cysteine in the nitrilase NitA from *Comamonas testosteroni* described in Fig. 1.

## Patentansprüche

1. Modifizierte Nitrilase, **dadurch gekennzeichnet, dass**:
a) die Aminosäure an Position 162 der Nitrilase von einem Cystein ersetzt wird, wobei sich das Cystein von der ursprünglichen Aminosäure der Nitrilase unterscheidet, wobei die Position der Aminosäuren im Verhältnis zur Primärsequenz der Nitrilase NitB von *Alcaligenes faecalis* wie auf der Figur 1 dargestellt definiert ist, und
b) die Selektivität der Nitrilase erhöht ist, wobei die Selektivität der Nitrilase als der Prozentsatz von durch die Hydrolyse eines von der Nitrilase katalysierten Nitrils freigesetzten Verbindungen bestimmt wird, die keine Carboxylfunktion besitzen,
mit Ausnahme der Nitrilase b_Gterrae von *Gordona terrae* (Zugangsnr. GeneBank: E12616) und der Nitrilase b_Afaecalis von *Alcaligenes faecalis* (Zugangsnr. SwissProt: P20960).

2. Modifizierte Nitrilase nach Anspruch 1, **dadurch gekennzeichnet, dass** die Nitrilase, in der die Aminosäure an Position 162 von einem Cystein ersetzt wird, ausgewählt ist aus den Nitrilasen p_Athalia 1 bis 4 von *Arabidopsis thaliana,* den Nitrilasen p_Tobacco 1 und 2 von *Nicotiana tabacum,* der Nitrilase b_Rhodoc J1 von *Rhodococcus rhodocrous* J1, der Nitrilase b_Rrhodoc PA3 von *Rhodococcus rhodocrous* PA34, der Nitrilase b_Rrhodoc K22 von *Rhodococcus rhodocrous* K 22, der Nitrilase b_Kozaenac von *Klebsiella ozaenae* und der Nitrilase Nit A von *Comamonas testosteroni,* die in der Figur 1 beschrieben sind.

3. Modifizierte Nitrilase nach einem der vorangehenden Ansprüche, **dadurch gekennzeichnet, dass** sie aus der in der Figur 1 beschriebenen Nitrilase NitA von *Comamonas testosteroni* besteht, in der die Aminosäure an Position 162 von einem Cystein ersetzt wird.

4. Nukleinsäuresequenz, insbesondere eine für eine nach einem der Ansprüche 1 bis 3 modifizierte Nitrilase codierende DNA-Sequenz.

5. Chimäres Gen oder Expressionskassette, **dadurch gekennzeichnet, dass** es in Transkriptionsrichtung eine funktionelle Promotorsequenz in einem Wirtsorganismus umfasst, wobei die Nukleinsäuresequenz nach Anspruch 4 für eine modifizierte Nitrilase codierend ist und eine funktionelle Terminatorsequenz im selben Wirtsorganismus.

6. Nicht humaner transformierter Wirtsorganismus, **dadurch gekennzeichnet, dass** er ein chimäres Gen nach Anspruch 5 umfasst, das stabil in seinem Genom integriert ist.

7. Wirtsorganismus nach Anspruch 6, **dadurch gekennzeichnet, dass** er aus den Bakterien, den Hefen, den Pilzen, den Pflanzenzellen und den Pflanzen ausgewählt ist.

8. Verfahren zur Herstellung modifizierter Nitrilasen nach einem der Ansprüche 1 bis 3, **dadurch gekennzeichnet, dass** es darin besteht, einen nach einem der Ansprüche 6 bis 7 transformierten Wirtsorganismus zu kultivieren und gegebenenfalls die modifizierte Nitrilase zu isolieren, im Gemisch oder in gereinigter Form.

9. Verwendung einer modifizierten Nitrilase nach einem der Ansprüche 1 bis 3 in einer Biokatalysereaktion in einem Syntheseverfahren oder zum Abbau chemischer Verbindungen.

10. Verwendung der modifizierten Nitrilase nach Anspruch 3 für die Hydrolyse von HMTBN oder von AMTBN.

11. Verfahren zur Erhöhung der Selektivität einer Nitrilase, wobei die Selektivität der Nitrilase definiert ist als der Prozentsatz von Verbindungen, die keine durch die Hydrolyse eines von der Nitrilase katalysierten Nitrils freigesetzte Carboxylfunktion besitzen, **dadurch gekennzeichnet, dass** es darin besteht, die Aminosäure an Position 162 der Nitrilase durch ein Cystein zu ersetzen, wobei sich das Cystein von der ursprünglichen Aminosäure der Nitrilase unterscheidet, wobei die Position der Aminosäuren im Verhältnis zur Primärsequenz der Nitrilase NitB von *Alcaligenes faecalis* wie auf der Figur 1 dargestellt definiert ist.

12. Verfahren nach Anspruch 11, **dadurch gekennzeichnet, dass** die Nitrilase, in der die Aminosäure an Position 162 von einem Cystein ersetzt wird, ausgewählt ist aus den Nitrilasen p_Athalia 1 bis 4 von *Arabidopsis thaliana,* den Nitrilasen p_Tobacco 1 und 2 von *Nicotiana tabacum,* der Nitrilase b_Rhodoc J1 von *Rhodococcus rhodocrous* J1, der Nitrilase b_Rrhodoc PA3 von *Rhodococcus rhodocrous* PA34, der Nitrilase b_Rrhodoc K22 von *Rhodococcus rhodocrous* K 22, der Nitrilase b_Kozaenac von *Klebsiella ozaenae* und der Nitrilase Nit A von *Comamonas testosteroni,* die in der Figur 1 beschrieben sind.

13. Verfahren nach Anspruch 12, **dadurch gekennzeichnet, dass** es darin besteht, die Aminosäure an Position 162 durch ein Cystein in der Nitrilase NitA von *Comamonas testosteroni* wie in Figur 1 beschrieben zu ersetzen.
